# EUROPEAN PATENT APPLICATION

(11) **EP 4 650 431 A1**
(43) Date of publication of application: **19.11.2025**
(21) Application number: 24744634.7
(22) Date of filing: 16.01.2024
(51) Int. Cl.: C12M 1/26, A61B 5/15

(54) **HOLDER AND SAMPLING KIT**

(30) Priority: 16.01.2023 JP 2023004449
(71) Applicant: Terumo Kabushiki Kaisha, Tokyo 151-0072 (JP)
(72) Inventor: OKABE, Hiroshi, Fujinomiya-shi, Shizuoka 418-0004 (JP)
(74) Representative: TBK
(86) International application number: PCT/JP2024/000888
(87) International publication number: WO 2024/154713

(57) **Abstract**

A holder (14) for connecting a culture bottle (16) to a collection kit (10) includes: a needle unit (18) including a needle tube (34) to be punctured into a mouth portion (16a) of the culture bottle (16) and a needle base portion (36) that supports the needle tube (34); a first tube (22) supported by the needle base portion (36) and including a first chamber (58) that accommodates the needle tube (34) therein; and a second tube (24) including a second chamber (66) that accommodates the mouth portion (16a) of the culture bottle (16) and movable relative to the first tube (22). The second tube (24) moves toward the needle unit (18) to cause the needle tube (34) to puncture into the mouth portion (16a) of the culture bottle (16).

## Description

### Technical Field

The present invention relates to a holder and a collection kit.

### Background Art

Blood preparations include red blood cell preparations, plasma preparations, platelet preparations, and whole blood preparations. As the blood preparation, a preparation containing components required by a patient is used for blood transfusion. The blood preparation is stored and transfused in a state of being accommodated in a medical bag. In order to ensure safety, a small amount of the blood preparation as a sample may be collected and subjected to a culture test. In the culture test, the sample is collected in a culture bottle, and the culture bottle is placed in an environment suitable for proliferation of bacteria to detect the presence or absence of pathogens.

For example, a platelet preparation is examined by the following procedure. A small volume collection bag is connected to a medical bag (hereinafter referred to as a platelet bag) accommodating the platelet preparation. Thereafter, a part of the platelet preparation in the platelet bag is transferred to the collection bag. Next, the collection bag is separated from the platelet bag.

Next, the collection bag is carried to a controlled environment such as a clean bench or a safety cabinet. In the clean bench or the safety cabinet, the platelet preparation in the collection bag is injected into a blood culture bottle. Prior to the injection, a tube of the collection bag is connected to a tube extending from a sample collection pipe. Using a scale of the sample collection pipe as a guide, a prescribed amount of the platelet preparation is transferred from the collection bag to the sample collection pipe.

Thereafter, an operator operates a valve of the sample collection pipe while visually observing the scale of the sample collection pipe, and transfers the prescribed amount of platelet preparation from the sample collection pipe to the blood culture bottle. In the culture test, anaerobic culture and aerobic culture are performed. Therefore, the platelet preparation is dispensed into a culture bottle used for the anaerobic culture and a culture bottle used for the aerobic culture. Thereafter, the culture bottle is set in a culture device and subjected to a culture test for a predetermined period.

For example, U.S. Pat. No. 8777921 discloses a collection kit for collecting a sample from a medical bag. The collection kit includes a holder for connecting a culture bottle to one end. The holder includes a tube that accommodates a mouth portion of the culture bottle, and a needle tube that is disposed inside the tube and is punctured into a rubber stopper of the culture bottle. The tube of the holder is covered with a lid. The lid is removed from the holder immediately before use. When the lid is removed, the needle tube of the holder is exposed. The mouth portion of the culture bottle is inserted into the tube of the holder and connected to the holder.

### Summary of Invention

In a holder of a collection kit in the related art, a needle tube extends to a portion close to an opening portion of a tube of the holder. Therefore, when a culture bottle is attached to the holder, the needle tube may come into contact with a hand of an operator or an unsterilized portion of the culture bottle, thereby contaminating the needle tube.

Therefore, there is a demand for a holder and a collection kit capable of preventing bacteria from adhering to a needle tube when a culture bottle is connected.

An object of the invention is to solve the above-described problems.
(1) A first aspect of the disclosure described below is a holder for connecting a culture bottle to a collection kit. The holder includes: a needle unit including a needle tube to be punctured into a mouth portion of the culture bottle and a needle base portion that supports the needle tube; a first tube supported by the needle base portion and including a first chamber that accommodates the needle tube therein; and a second tube including a second chamber that accommodates the mouth portion of the culture bottle and movable relative to the first tube. The second tube moves toward the needle unit to cause the needle tube to puncture into the mouth portion of the culture bottle.
   In the holder of the item (1), the needle tube is disposed in the first tube that is deeper than the second tube that accommodates the mouth portion of the culture bottle. Therefore, the holder can prevent contact between the needle tube and a hand of an operator, an unsterilized portion of the culture bottle, or the like when the culture bottle is connected.
(2) The holder according to item (1) may further include a sealing member that seals a gap between the first tube and the second tube and prevents bacteria from entering the first chamber.
   The holder of item (2) can prevent entry of bacteria through the gap between the first tube and the second tube, and can prevent contamination of the needle tube.
(3) The holder according to item (1) or (2) may further include a partition wall provided in the second tube to partition the first chamber and the second chamber.
   The holder of item (3) can more effectively prevent contamination of the needle tube by further reducing exposure of the needle tube by the partition wall.
(4) In the holder according to item (3), the partition wall may have a through-hole through which the needle tube passes.
   The holder of item (4) can easily puncture the needle tube into the mouth portion of the culture bottle through the through-hole while preventing exposure of the needle tube by the partition wall. In addition, since the needle tube does not come into contact with a portion other than a rubber stopper of the culture bottle, contamination of the needle tube can be more effectively prevented.
(5) In the holder according to item (4), the needle tube may be located on a proximal side with respect to the through-hole of the partition wall in an initial state before the culture bottle is attached.
   The holder of item (5) can prevent contamination of the needle tube by preventing the needle tube from protruding from the partition wall.
(6) In the holder according to any one of items (1) to (5), the second tube may include an engagement protrusion that engages with the mouth portion of the culture bottle accommodated in the second chamber to prevent the culture bottle from being detached from the second tube.
   The holder of item (6) can prevent the culture bottle from being detached from the second tube due to an elastic repulsive force of the rubber stopper sealing the mouth portion of the culture bottle. Accordingly, operability is improved, and a large number of culture bottles can be efficiently connected.
(7) In the holder according to item (2), the second tube may include a sliding portion that slides while overlapping the first tube in a radial direction, and the sealing member may include a packing that seals the sliding portion.
   The holder of item (7) can move the second tube with respect to the first tube while preventing contamination of the first chamber.
(8) In the holder according to item (2), the sealing member may include an elastic tube body made of an elastic material that is deflatable in an axial direction, and the elastic tube body may be closely attached to the first tube and the second tube, and the first tube and the second tube are connected via the elastic tube body.
   The holder of item (8) can advance the second tube toward the needle tube by deforming the elastic tube body, and can puncture the needle tube into the mouth portion of the culture bottle while preventing contamination of the first chamber.
(9) The holder according to item (8) may further include a first guide portion provided in the first tube and protruding toward the second tube, and a second guide portion provided in the second tube, protruding toward the first tube, and sliding on the first guide portion. The first guide portion and the second guide portion may guide a displacement direction of the second tube in the axial direction.
   The holder of item (9) prevents axial misalignment of the second tube and facilitates puncture of the needle tube into the mouth portion of the culture bottle.
(10) The holder according to any one of items (1) to (9) may further include a lid that covers an opening portion of the second tube. The lid may include a plate body that covers the opening portion and a fitting wall that is fitted to the second tube to prevent bacteria from entering the first chamber and the second chamber.
   The holder of item (10) can prevent bacteria from entering from the opening portion of the second tube.
(11) The holder according to any one of items (1) to (9) may further include a lid that covers an opening portion of the second tube. The lid may include a sheet body that prevents passage of bacteria, and a joint portion that joints the sheet body to the second tube.
   The holder of item (11) can prevent bacteria from entering from the opening portion of the second tube.
(12) In the holder according to any one of items (1) to (11), the needle base portion may include a main body portion extending in an axial direction and jointed to an outer peripheral portion of the needle tube, a flange protruding outward in a plate shape from the main body portion, and a first screw formed in the main body portion. The first tube may be screwed to the first screw and attached to the flange.
   The holder of item (12) can fix the first tube to the needle base portion and prevent bacteria from entering the first chamber.
(13) In the holder according to any one of items (1) to (11), the needle base portion may include a main body portion extending in an axial direction and jointed to an outer peripheral portion of the needle tube, and a flange protruding outward in a plate shape from the main body portion. The first tube may be jointed to the flange.
   The holder of item (13) can prevent contamination of the first chamber by jointing the first tube and the needle base portion without a gap.
(14) A second aspect of the disclosure described below is a collection kit that includes: a liquid accommodation unit that accommodates a liquid; and a holder that communicates with the liquid accommodation unit and to which a culture bottle is connected. The holder includes a needle unit including a needle tube to be punctured into a mouth portion of the culture bottle and a needle base portion that supports the needle tube, a first tube supported by the needle base portion and including a first chamber that accommodates the needle tube therein, and a second tube including a second chamber that accommodates the mouth portion of the culture bottle and movable relative to the first tube. The second tube moves toward the needle unit to cause the needle tube to puncture into the mouth portion of the culture bottle.

The collection kit of item (14) can connect the culture bottle to the collection kit while preventing contamination of the liquid.

### Brief Description of Drawings

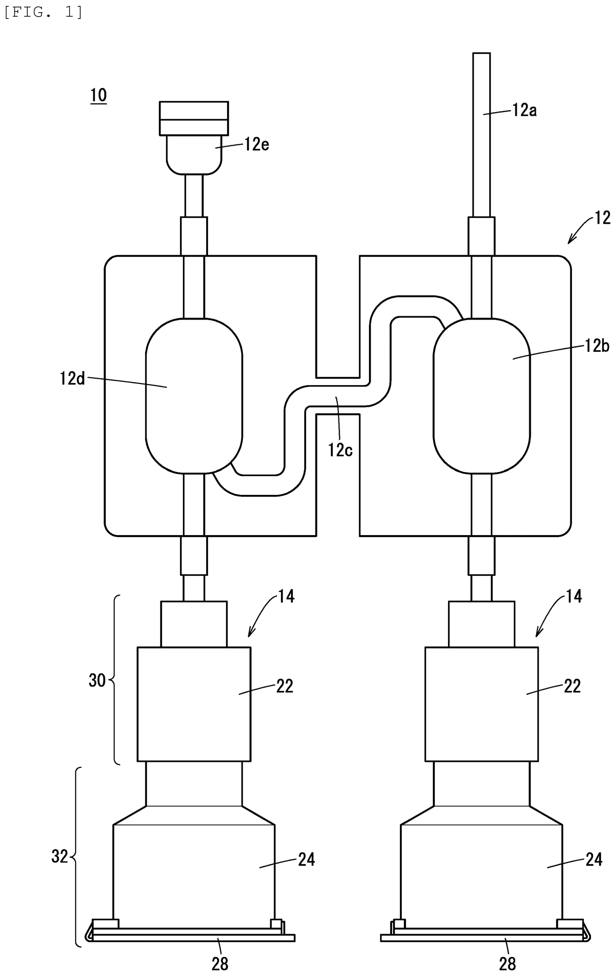

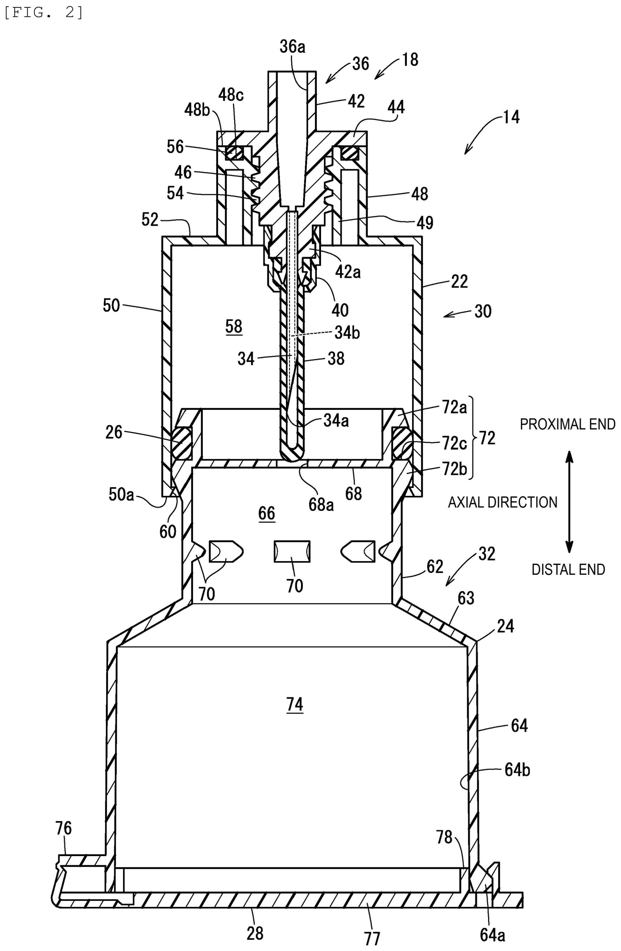

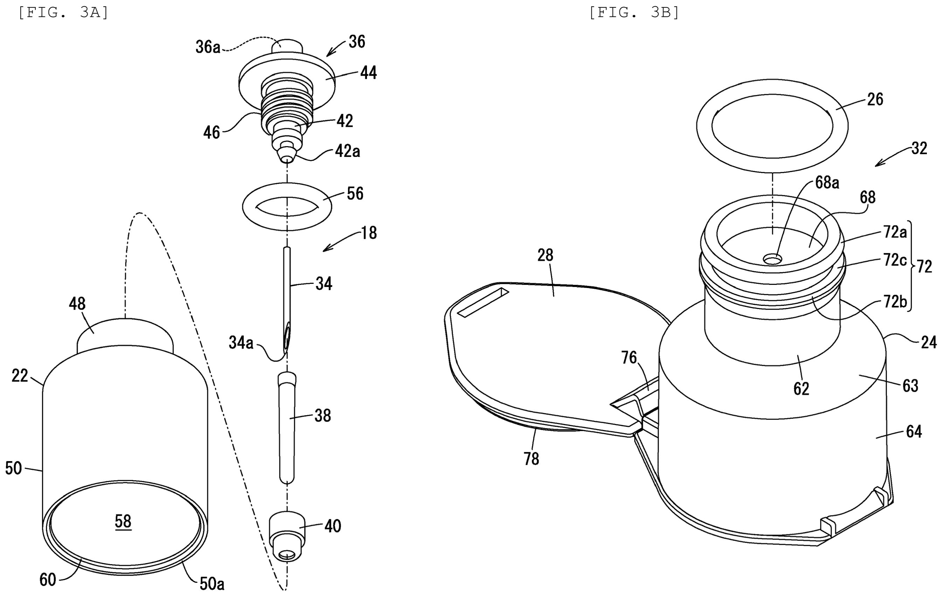

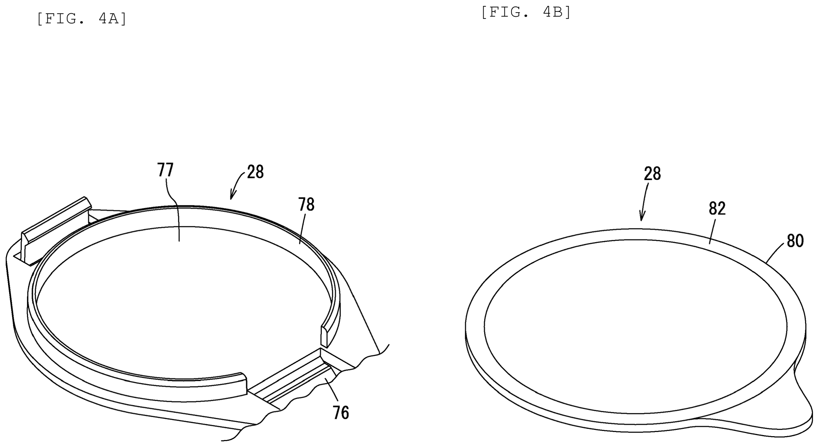

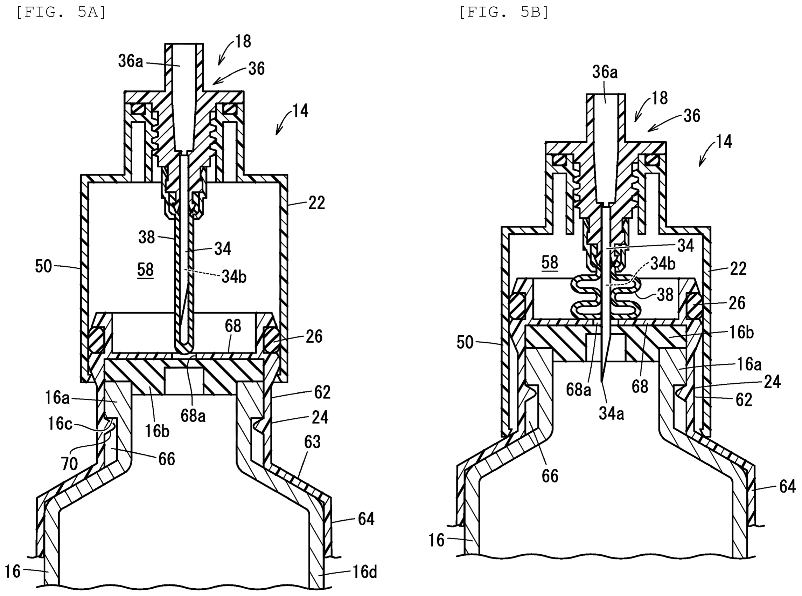

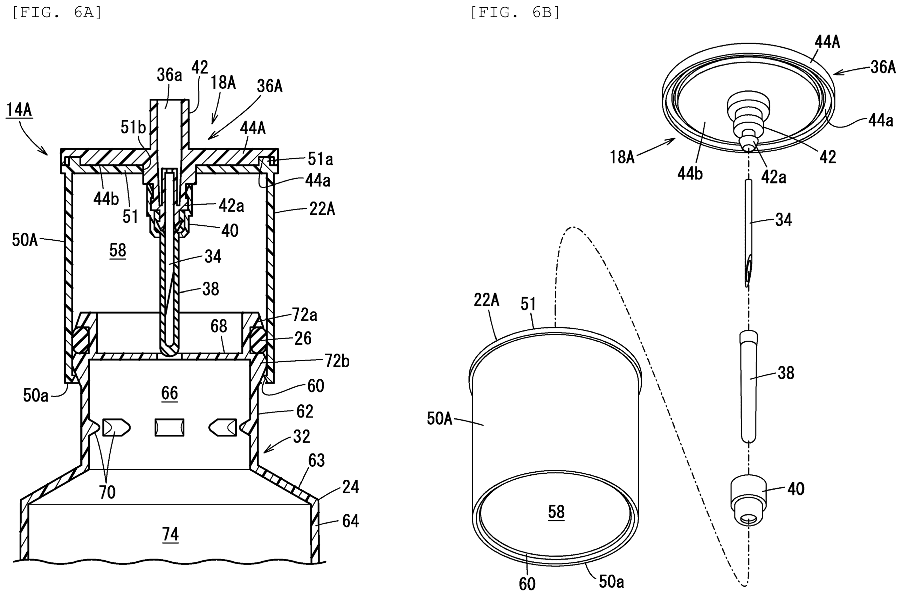

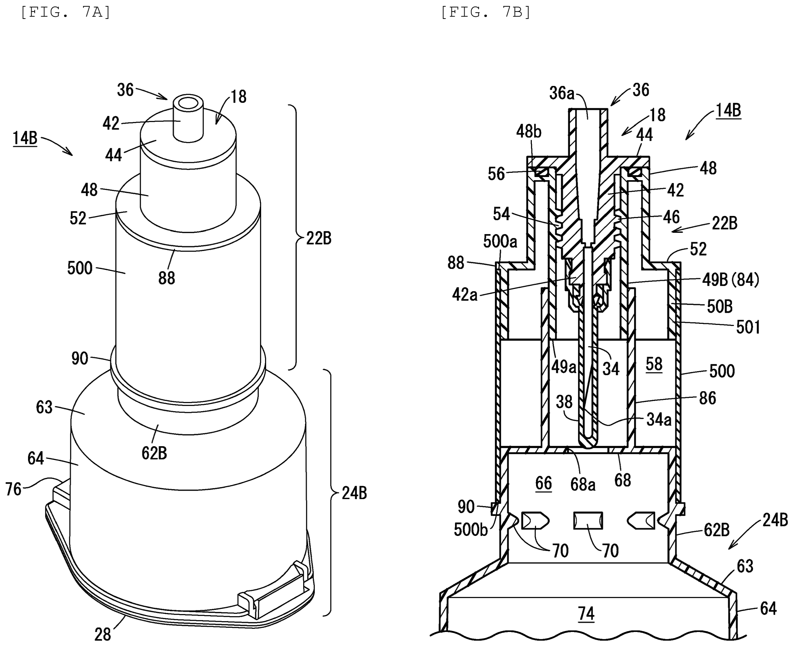

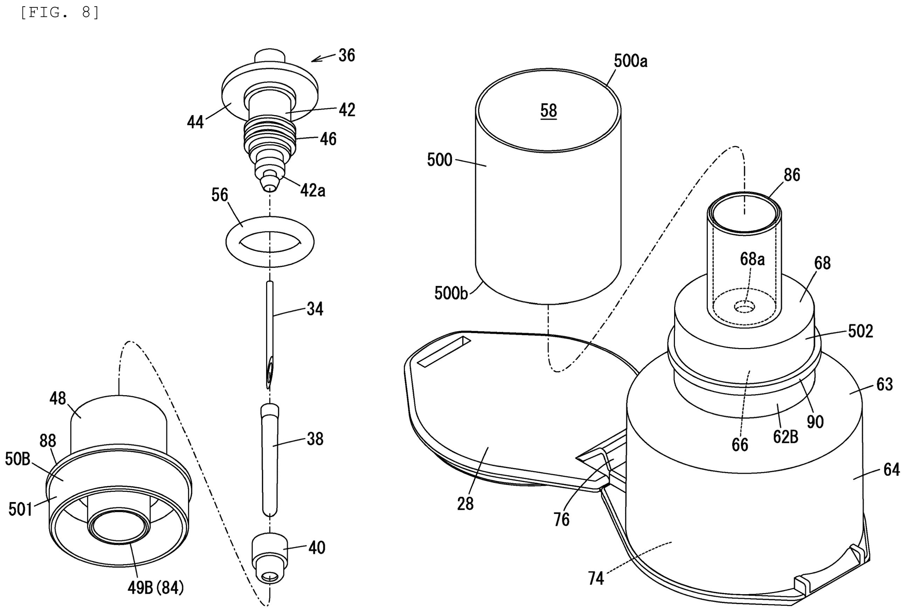

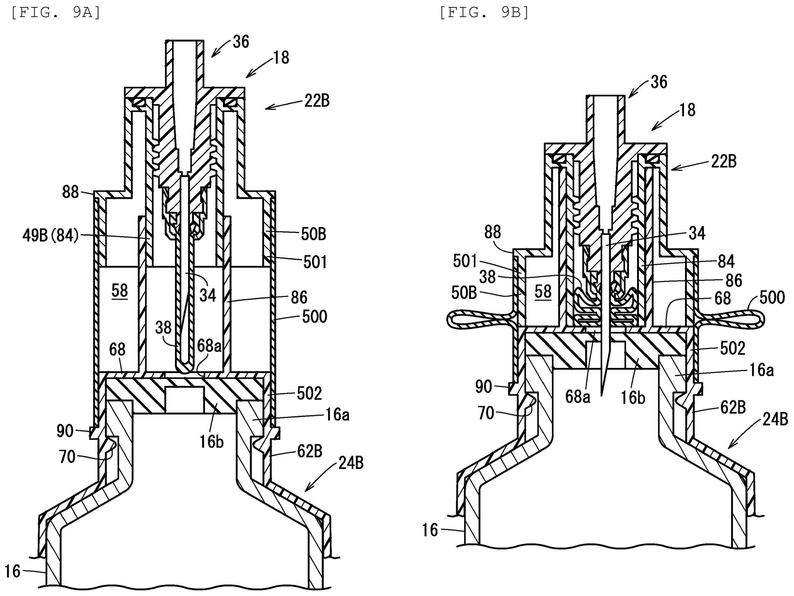

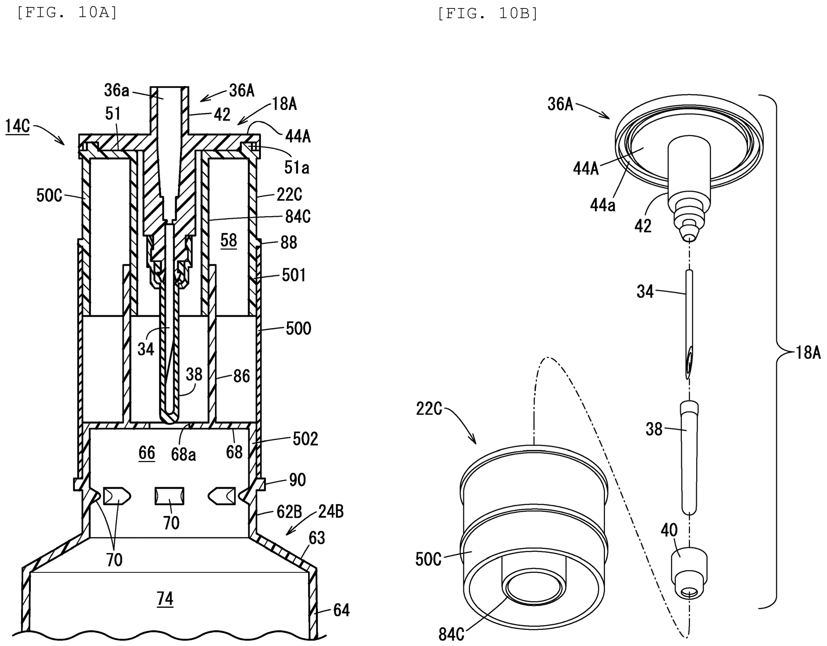

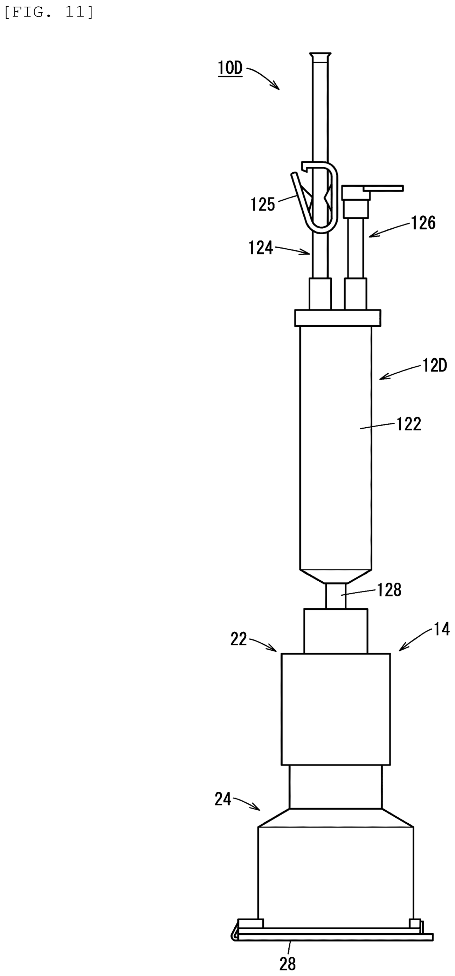

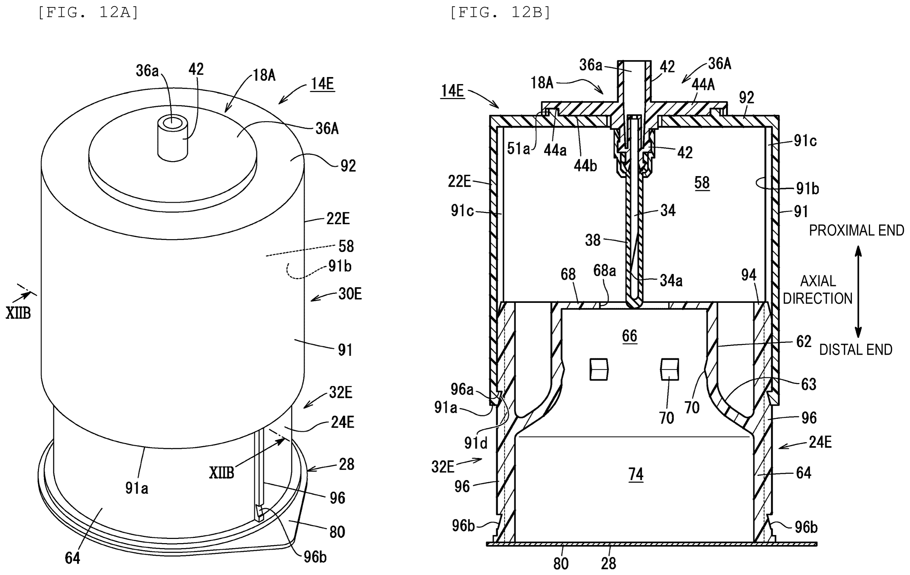

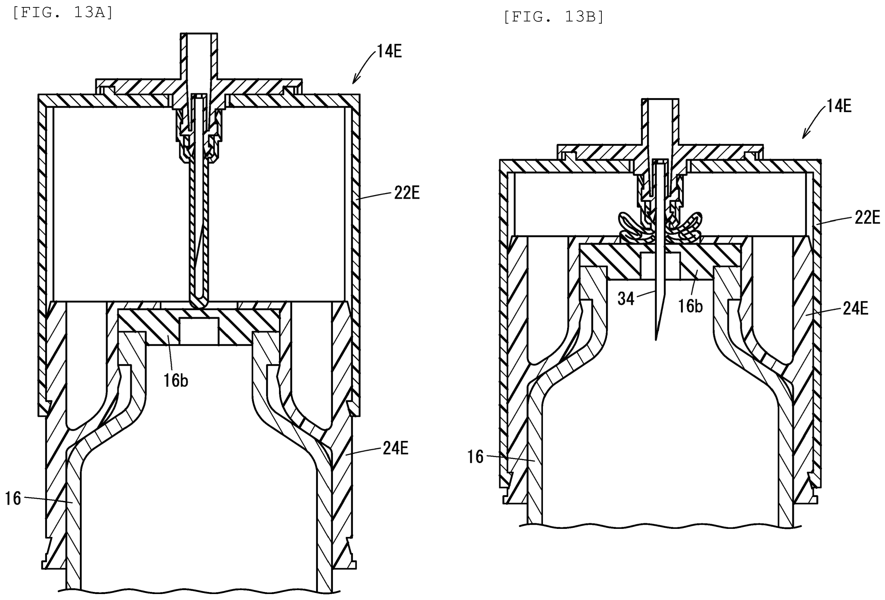

Fig. 1 is a plan view of a collection kit according to a first embodiment. Fig. 2 is a cross-sectional view of a holder of Fig. 1. Fig. 3A is an exploded perspective view of a needle unit and a first tube of Fig. 2, and Fig. 3B is an exploded perspective view of a second tube of Fig. 2. Fig. 4A is a perspective view showing a first configuration example of a lid, and Fig. 4B is a perspective view showing a second configuration example of the lid. Fig. 5A is a cross-sectional view of a state in which a culture bottle is attached to the second tube of the holder of Fig. 2, and Fig. 5B is a cross-sectional view of a state in which the second tube of Fig. 5A is pushed toward the first tube and a needle tube is punctured into a rubber stopper of the culture bottle. Fig. 6A is a cross-sectional view of a holder according to a second embodiment, and Fig. 6B is an exploded perspective view of a needle unit and a first tube of Fig. 6A. Fig. 7A is a perspective view of a holder according to a third embodiment, and Fig. 7B is a cross-sectional view of the holder of Fig. 7A. Fig. 8 is an exploded perspective view of the holder of Fig. 7A. Fig. 9A is a cross-sectional view of a state in which a culture bottle is connected to a second tube of the holder of Fig. 7A, and Fig. 9B is a cross-sectional view of a state in which the second tube of Fig. 9A is pushed toward a first tube and a needle tube is punctured into a rubber stopper of the culture bottle. Fig. 10A is a cross-sectional view of a holder according to a fourth embodiment, and Fig. 10B is an exploded perspective view of a needle unit and a first tube of Fig. 10A. Fig. 11 is a plan view of a collection kit according to a fifth embodiment. Fig. 12A is a perspective view of a holder according to a sixth embodiment, and Fig. 12B is a cross-sectional view taken along a line XIIB-XIIB of Fig. 12A. Fig. 13A is a cross-sectional view of a state in which a culture bottle is connected to a second tube of the holder of Fig. 12A, and Fig. 13B is a cross-sectional view of a state in which the second tube of Fig. 13A is pushed toward a first tube and a needle tube is punctured into a rubber stopper of the culture bottle.

### Description of Embodiments

### (First Embodiment)

As shown in Fig. 1, a collection kit 10 is used to measure a prescribed amount of a liquid and dispense the liquid into two culture bottles 16 (see Fig. 5A) when performing a culture test of a blood preparation. The collection kit 10 includes holders 14 and a liquid accommodation unit 12 that accommodates the liquid.

The liquid accommodation unit 12 includes a connection tube 12a, a first sample chamber 12b, a second sample chamber 12d, a connection flow path 12c, and an exhaust port 12e. The liquid accommodation unit 12 has a structure in which two flexible resin sheets are overlapped and peripheral edge portions of the connection tube 12a, the first sample chamber 12b, and the second sample chamber 12d are welded. The connection tube 12a is a tube extending from an upper end portion of the liquid accommodation unit 12. The connection tube 12a is used to introduce the liquid (blood preparation) from a preparation bag (not shown) accommodating a blood preparation to be tested into the liquid accommodation unit 12.

The first sample chamber 12b and the second sample chamber 12d have a predetermined volume of, for example, 8 ml or 10 ml. The first sample chamber 12b and the second sample chamber 12d accommodate a predetermined amount of the liquid determined in the culture test. The first sample chamber 12b communicates with the connection tube 12a. The second sample chamber 12d communicates with the second sample chamber 12d via the connection flow path 12c. A periphery of the connection flow path 12c is cut, and the first sample chamber 12b and the second sample chamber 12d can be separated by a closing member such as a clamp. The exhaust port 12e is connected to an upper end of the second sample chamber 12d. The exhaust port 12e includes a fiber filter that blocks inflow of bacteria. The exhaust port 12e discharges air inside the liquid accommodation unit 12.

The holder 14 is connected to a lower portion of the first sample chamber 12b and a lower portion of the second sample chamber 12d. The holder 14 connects the culture bottle 16 and the collection kit 10. One holder 14 is used to introduce the liquid in the first sample chamber 12b into the first culture bottle 16. The other holder 14 is used to introduce the liquid in the second sample chamber 12d into the culture bottle 16. One culture bottle 16 is used for a culture test under anaerobic conditions. The other culture bottle 16 is used for a culture test under aerobic conditions.

The culture test includes a culture step of disposing the culture bottle 16 in a thermostatic chamber for a predetermined time. When the liquid contains bacteria, the bacteria are detected in the subsequent test step. Since such a culture test has high sensitivity, when the liquid is dispensed into the culture bottle 16, if even a small amount of bacteria is mixed into the liquid, a detection result (false positive) indicating that bacteria are contained is generated. In this case, the valuable platelet preparation is discarded. In order to prevent such waste of the platelet preparation, the collection kit 10 according to the embodiment employs the holder 14 capable of preventing contamination of the liquid.

As shown in Fig. 2, the holder 14 includes a needle unit 18, a first tube 22, a second tube 24, a packing 26 (sealing member), and a lid 28. The needle unit 18 and the first tube 22 are integrally jointed to each other, and constitute one component as a first module 30. In addition, the second tube 24, the packing 26, and the lid 28 constitute a second module 32.

As shown in Fig. 3A, the needle unit 18 includes a needle tube 34, a needle base portion 36, a needle cover 38, and a retaining ring 40. The needle tube 34 has a sharp needle tip 34a. The needle tube 34 extends toward a distal side in an axial direction along a central axis of the holder 14. The needle tube 34 has a flow path 34b through which the liquid flows. As shown in Fig. 2, the flow path 34b penetrates the needle tube 34 in the axial direction and communicates with a hollow portion 36a of the needle base portion 36.

As shown in Fig. 3A, the needle base portion 36 includes a main body portion 42 that extends in a tubular shape in the axial direction. The main body portion 42 includes a needle holding portion 42a on the distal side. The needle holding portion 42a is jointed to the needle tube 34 and supports the needle tube 34. The needle base portion 36 supports the needle tube 34 by the main body portion 42. The needle base portion 36 includes the hollow portion 36a inside the main body portion 42. The hollow portion 36a extends in the axial direction and opens on a proximal side. The proximal side of the needle base portion 36 is jointed to the liquid accommodation unit 12. The hollow portion 36a communicates with the first sample chamber 12b (or the second sample chamber 12d).

The needle base portion 36 further includes a flange 44 and a first screw 46. The flange 44 protrudes outward in a radial direction in a disc shape from the main body portion 42. The flange 44 is located near a center of the main body portion 42 in the axial direction. The first screw 46 is a screw thread or a screw groove formed on an outer peripheral surface of the main body portion 42. The first screw 46 is provided on the distal side of the flange 44. A second screw 54 of the first tube 22 is screwed to the first screw 46.

The needle cover 38 is a tubular member formed of a flexible material such as rubber or elastomer. The distal side of the needle cover 38 is closed. The needle cover 38 protects the needle tube 34 from contamination by covering the outside of the needle tube 34. A proximal end of the needle cover 38 is attached to an outer peripheral portion of the needle holding portion 42a of the needle base portion 36. The retaining ring 40 is attached to the outer peripheral portion of the needle holding portion 42a. The retaining ring 40 is fitted to an outer side of the needle holding portion 42a so as to sandwich the needle cover 38, and fixes the needle cover 38 to the needle holding portion 42a.

The first tube 22 is a tubular member and is jointed to the flange 44. As shown in Fig. 3A, the first tube 22 includes a cylindrical small-diameter portion 48 and a cylindrical large-diameter portion 50. The small-diameter portion 48 is located on the proximal side, and the large-diameter portion 50 is located on the distal side. The small-diameter portion 48 and the large-diameter portion 50 are integrally connected via a step portion 52. The small-diameter portion 48 has an outer diameter dimension the same as an outer diameter of the flange 44.

As shown in Fig. 2, the small-diameter portion 48 includes the second screw 54 and a packing 56. The second screw 54 is located on an inner peripheral surface of a connection tube 49 extending in a tubular shape inside the small-diameter portion 48. The second screw 54 has a screw thread or a screw groove. The small-diameter portion 48 is fixed to the needle base portion 36 by screwing the second screw 54 to the first screw 46. A proximal portion 48b of the small-diameter portion 48 is attached to the flange 44. The packing 56 is attached to the proximal portion 48b to seal a gap between the small-diameter portion 48 and the flange 44. The packing 56 is a ring-shaped elastic material. The packing 56 is held in a packing accommodation groove 48c of the proximal portion 48b.

The large-diameter portion 50 has a cylindrical shape extending in the axial direction. A distal portion 50a of the large-diameter portion 50 is located on the distal side with respect to the needle tube 34 and the needle cover 38. The large-diameter portion 50 forms a first chamber 58 that accommodates the needle tube 34 therein. As shown in Fig. 5A, an inner diameter of the large-diameter portion 50 is larger than an outer diameter of a mouth portion 16a of the culture bottle 16, and has a dimension capable of accommodating a head portion 62 of the second tube 24. As shown in Fig. 2, the large-diameter portion 50 includes a rib 60 that prevents the second tube 24 from being pulled out on an inner peripheral surface near the distal portion 50a. The rib 60 extends annularly over the entire region in a circumferential direction.

As shown in Figs. 2 and 3B, the second tube 24 of the second module 32 is a tubular member including the head portion 62 and a body portion 64. The head portion 62 is located on the proximal side of the second tube 24. The head portion 62 has an outer diameter and an inner diameter smaller than those of the body portion 64. The head portion 62 includes a second chamber 66, a partition wall 68, and a claw portion 70 therein. The second chamber 66 accommodates the mouth portion 16a of the culture bottle 16. A proximal end of the second chamber 66 is partitioned by the partition wall 68. The distal side of the second chamber 66 opens toward a third chamber 74 of the body portion 64.

The partition wall 68 is a wall-shaped member protruding inward of the head portion 62. The partition wall 68 covers the distal side of the first chamber 58 of the first tube 22 in a state in which the second tube 24 is attached to the first tube 22. The partition wall 68 partitions the first chamber 58 that accommodates the needle tube 34 and the second chamber 66 that accommodates the mouth portion 16a of the culture bottle 16. The partition wall 68 prevents contamination of the needle tube 34 and the needle cover 38 by preventing exposure of the needle tube 34 and the needle cover 38. The partition wall 68 has a through-hole 68a at a center thereof. The through-hole 68a has an inner diameter dimension that allows the needle tube 34 and the needle cover 38 to pass therethrough without contacting the partition wall 68. The through-hole 68a prevents contamination of the needle tube 34 and the needle cover 38 by preventing the needle tube 34 and the needle cover 38 from contacting the partition wall 68 that may be contaminated.

As shown in Fig. 2, a plurality of claw portions 70 are provided. The claw portion 70 protrudes inward from an inner peripheral surface of the head portion 62. The plurality of claw portions 70 have a shape in which an annular protrusion is divided at a plurality of positions in the circumferential direction. The plurality of claw portions 70 are fitted to unevenness of the mouth portion 16a of the culture bottle 16 to prevent the mouth portion 16a from being detached from the second chamber 66.

The head portion 62 has a first rib 72a and a second rib 72b on an outer peripheral portion. The first rib 72a and the second rib 72b are annular protrusions protruding outward from the head portion 62. The second tube 24 includes the first rib 72a and the second rib 72b as a sliding portion 72 that slides on the first tube 22. The second rib 72b is located on the distal side and comes into contact with the rib 60 of the first tube 22 to prevent the second tube 24 from falling off the first tube 22.

The first rib 72a and the second rib 72b are separated from each other in the axial direction. The sliding portion 72 has an accommodation groove 72c between the first rib 72a and the second rib 72b. The accommodation groove 72c accommodates the ring-shaped packing 26. The packing 26 slidably comes into contact with the inner peripheral surface of the large-diameter portion 50 of the first tube 22. The packing 26 prevents a fluid from moving along a gap between the first tube 22 and the second tube 24. The packing 26 prevents contamination of the needle tube 34 by preventing foreign matter and bacteria from entering the first chamber 58.

The body portion 64 is located on the distal side of the head portion 62. The body portion 64 is integrally connected to the head portion 62 via a shoulder portion 63. The body portion 64 has a cylindrical shape and has the third chamber 74 surrounded by an inner peripheral surface 64b therein. The third chamber 74 accommodates a main body 16d of the culture bottle 16 (see Fig. 5A). The third chamber 74 opens at a distal portion 64a of the body portion 64. In order to prevent contamination during storage and transportation, the third chamber 74 is closed by the lid 28 in an initial state provided as a product.

The lid 28 is connected to the second tube 24 via a hinge 76. The lid 28 can be displaced from a closed position where the lid 28 closes the third chamber 74 of Fig. 2 to an open position where the lid 28 opens the third chamber 74 of Fig. 3B when the lid 28 can move rotationally about the hinge 76. As shown in Fig. 4A, the lid 28 includes a plate body 77 and a fitting wall 78 protruding from the plate body 77. The fitting wall 78 is fitted to the inner peripheral surface 64b of the body portion 64 of the second tube 24. The fitting wall 78 is in close contact with the inner peripheral surface 64b to prevent bacteria from entering the inside of the third chamber 74.

Note that the lid 28 may include a packing that is mounted on the fitting wall 78 and closes a gap between the fitting wall 78 and the inner peripheral surface 64b. In addition, the lid 28 may include a packing disposed between the plate body 77 and the distal portion 64a of the body portion 64.

Note that the lid 28 may be a sheet body 80 shown in Fig. 4B. As the sheet body 80, a sheet-like material having air permeability and flexibility for preventing entry of bacteria can be used. By using a material having air permeability, it is possible to perform sterilization treatment using a gas or air such as water vapor in a state in which the second tube 24 is blocked by the sheet body 80. Examples of such a material include paper, a nonwoven fabric sheet, or the like. For example, tyvek (registered trademark) can be used. The sheet body 80 has a circular shape and has a dimension capable of closing the third chamber 74 (see Fig. 2). A joint portion 82 is provided at a peripheral edge portion of the sheet body 80. The joint portion 82 is bonded or welded to the distal portion 64a of the second tube 24. The lid 28 formed of the sheet body 80 is opened by peeling the joint portion 82 from the second tube 24 immediately before use.

The collection kit 10 according to the embodiment is configured as described above. Hereinafter, the collection kit 10 and the holder 14 are used as follows.

In the culture test, the liquid accommodation unit 12 of the collection kit 10 is filled with a liquid such as a platelet preparation. The same amount of the liquid (for example, 8 ml or 10 ml) is accommodated in the first sample chamber 12b and the second sample chamber 12d. Such a collection kit 10 is carried into a clean bench prior to dispensing. Next, the lid 28 of the holder 14 is opened.

Next, a rubber stopper 16b of the mouth portion 16a of the culture bottle 16 is performed a sterilization treatment in the clean bench. The sterilization treatment is performed by wiping a surface of the rubber stopper 16b with a disinfectant.

Next, as shown in Fig. 5A, the culture bottle 16 is connected to the holder 14. In this step, the mouth portion 16a of the culture bottle 16 is accommodated in the second chamber 66 of the second tube 24. When the culture bottle 16 is pushed toward the second tube 24 in a state in which the second tube 24 is supported, the mouth portion 16a rides over the claw portion 70 and is accommodated in the second chamber 66. The claw portion 70 engages with a step 16c (unevenness) of the mouth portion 16a to prevent the culture bottle 16 from being detached from the second tube 24.

The culture bottles 16 are respectively connected to one holder 14 and the other holder 14 of the collection kit 10. In this step, the needle tube 34 is located at a position largely retracted from the distal portion 64a of the second tube 24 to the proximal side. Further, the needle tube 34 and the needle cover 38 are covered by the partition wall 68. Therefore, the holder 14 can effectively prevent the needle tube 34 and the needle cover 38 from coming into contact with a hand of an operator or an unsterilized portion of the holder 14 until the culture bottle 16 is attached after the lid 28 of the holder 14 is opened.

Thereafter, the collection kit 10 and the culture bottle 16 are attached to a jig that pushes the holder 14 toward the culture bottle 16. The jig includes an upper holding portion that holds the first tube 22 of the holder 14 and a support table that supports the culture bottle 16. The jig includes an operation lever that displaces the upper holding portion toward the support table. Note that in the test step, the use of the jig is not essential, and the test step may be manually performed by the operator.

Next, the connection flow path 12c of the liquid accommodation unit 12 is closed, and the first sample chamber 12b and the second sample chamber 12d are fluidly separated.

Next, a user operates the operation lever of the jig to push the holder 14 into the culture bottle 16. As shown in Fig. 5B, the head portion 62 of the second tube 24 slides inside the first tube 22 toward the proximal end. The needle tube 34 and the needle cover 38 are inserted into the second chamber 66 through the through-hole 68a of the partition wall 68. The needle tube 34 penetrates the needle cover 38 and punctures the rubber stopper 16b. When the holder 14 is further pressed, the needle tube 34 penetrates the rubber stopper 16b, and the flow path 34b communicates with the inside of the culture bottle 16.

The inside of the culture bottle 16 is in a negative pressure state. Therefore, when the needle tube 34 penetrates the rubber stopper 16b, the liquid in the liquid accommodation unit 12 is sucked out to the culture bottle 16. Accordingly, a predetermined amount of the liquid is dispensed into the culture bottle 16.

As described above, in the holder 14, the needle tube 34 and the needle cover 38 puncture the rubber stopper 16b without touching portions other than the rubber stopper 16b. Therefore, the holder 14 can effectively prevent contamination of the liquid.

### (Second Embodiment)

As shown in Figs. 6A and 6B, a holder 14A according to the embodiment is different from the holder 14 of Fig. 2 in a needle unit 18A and a first tube 22A. Note that in the configuration of the holder 14A of Fig. 6A, the same components as those of the holder 14 of Fig. 2 are denoted by the same reference numerals, and detailed description thereof will be omitted.

As shown in Fig. 6A, the holder 14A includes the first tube 22A and a needle base portion 36A. The first tube 22A has a cylindrical large-diameter portion 50A and a proximal wall 51 that closes a proximal end of the large-diameter portion 50A. The large-diameter portion 50A has a cylindrical shape extending in the axial direction, and accommodates the head portion 62 of the second tube 24 so as to be slidable in the axial direction. The large-diameter portion 50A has the first chamber 58 therein.

The proximal wall 51 is configured as a wall surface that closes the proximal end of the large-diameter portion 50A. The proximal wall 51 has an engagement protrusion 51a and an attachment hole 51b. The engagement protrusion 51a is an annular protrusion that protrudes short from the proximal wall 51 toward the proximal side. The attachment hole 51b is located at a center of the proximal wall 51, and the main body portion 42 of the needle base portion 36A penetrates the attachment hole 51b. The proximal wall 51 is jointed to a flange 44A of the needle base portion 36A by a method such as welding or bonding.

The needle base portion 36A includes the flange 44A having a diameter the same as the large-diameter portion 50A. A groove portion 44a is formed in a distal portion 44b of the flange 44A. The engagement protrusion 51a engages with the groove portion 44a.

Other configurations of the holder 14A are the same as those of the holder 14 of Fig. 2. The holder 14A according to the embodiment can reduce the number of components, simplify the structure, and reduce the number of steps required for assembly by directly jointing the first tube 22A and the needle base portion 36A.

### (Third Embodiment)

As shown in Figs. 7A and 7B, a holder 14B according to the embodiment includes the needle unit 18, a first tube 22B, a second tube 24B, and the lid 28. Note that in the configuration of the holder 14B, the same components as those of the holder 14 of Fig. 2 are denoted by the same reference numerals, and detailed description thereof will be omitted.

As shown in Figs. 7B and 8, the first tube 22B mainly includes the small-diameter portion 48, a connection tube 49B, the step portion 52, a large-diameter portion 50B, and an elastic tube body 500. The connection tube 49B is a tubular member disposed inside the small-diameter portion 48 and extends toward the distal side. The connection tube 49B protrudes to the distal side from the needle holding portion 42a. However, a distal end 49a of the connection tube 49B is located on the proximal side with respect to the needle tip 34a of the needle tube 34. The connection tube 49B constitutes a first guide portion 84 that restricts a displacement direction of the second tube 24B in the axial direction.

The large-diameter portion 50B is located on the distal side of the step portion 52 and has a tubular shape extending short in the axial direction. An outer diameter dimension of the large-diameter portion 50B is the same as an outer diameter dimension of a head portion 62B of the second tube 24B. The large-diameter portion 50B includes a first adhesion portion 501 on an outer peripheral portion. The elastic tube body 500 is attached to the first adhesion portion 501. The first adhesion portion 501 is in close contact with the elastic tube body 500 without a gap and prevents bacteria from entering the first chamber 58.

The step portion 52 includes a first stopper 88 protruding outward in the radial direction. The first stopper 88 is attached to a proximal portion 500a of the elastic tube body 500 to prevent the elastic tube body 500 from moving in a proximal direction.

The elastic tube body 500 is a tubular member formed of an elastic material. The elastic tube body 500 extends in the axial direction, and a distal portion 500b thereof is located on the distal side with respect to the needle tube 34 and the needle cover 38. A part of the distal side of the elastic tube body 500 is mounted on the head portion 62B of the second tube 24B. The elastic tube body 500 is attached to the large-diameter portion 50B and forms, together with the large-diameter portion 50B, the first chamber 58 that accommodates the needle tube 34. In addition, the elastic tube body 500 also serves as a sealing member that seals a gap between the first tube 22B and the second tube 24B.

The head portion 62B of the second tube 24B is different from the second tube 24 of Fig. 2. The head portion 62B includes a second adhesion portion 502 to which the elastic tube body 500 is attached on an outer peripheral portion thereof. The second adhesion portion 502 is in close contact with the elastic tube body 500 without a gap and prevents bacteria from entering the first chamber 58. The head portion 62B includes a second stopper 90 protruding toward an outer periphery. The second stopper 90 is a rib-shaped protrusion extending in the circumferential direction. The second stopper 90 is attached to the distal portion 500b of the elastic tube body 500 to prevent the elastic tube body 500 from moving toward the distal side.

In addition, the head portion 62B includes a second guide portion 86 extending from the partition wall 68 in the proximal direction. The second guide portion 86 has a cylindrical shape. The second guide portion 86 accommodates the first guide portion 84 (the connection tube 49B) in an inner peripheral portion thereof. The second guide portion 86 can be displaced in the axial direction with respect to the first guide portion 84 while sliding on the first guide portion 84. The second guide portion 86 prevents displacement of the second tube 24B in the radial direction and guides a movement direction of the second tube 24B in the axial direction.

The holder 14B according to the embodiment operates as follows.

In an initial state of Fig. 7A, the second tube 24B is closed by the lid 28. In addition, the first chamber 58 that accommodates the needle tube 34 is sealed by the elastic tube body 500. Accordingly, the holder 14B can prevent contamination of the needle tube 34.

Next, the lid 28 of the holder 14B is opened. Thereafter, as shown in Fig. 9A, the culture bottle 16 is attached to the second tube 24B of the holder 14B. When the culture bottle 16 is attached, the partition wall 68 covers the needle tube 34 and the needle cover 38. Therefore, the holder 14B can prevent contamination of the needle tube 34 and the needle cover 38.

Next, as shown in Fig. 9B, the second tube 24B of Fig. 9A is pushed toward the first tube 22B in the axial direction. The elastic tube body 500 is deformed to bulge outward while being in close contact with the first adhesion portion 501 and the second adhesion portion 502. Therefore, the holder 14B can displace the second tube 24B toward the proximal end while maintaining the sealing of the first chamber 58. The second tube 24B is displaced toward the proximal end while being guided in the axial direction by the first guide portion 84 and the second guide portion 86. Therefore, the needle tube 34 and the needle cover 38 pass through the through-hole 68a without touching the partition wall 68. Therefore, the holder 14B can dispense the liquid into the culture bottle 16 while preventing contamination of the needle tube 34 and the needle cover 38.

### (Fourth Embodiment)

A holder 14C according to the embodiment shown in Fig. 10A includes a first tube 22C. Note that in the holder 14C, the needle unit 18A is configured similarly to the needle unit 18A described with reference to Fig. 6A. In addition, the second tube 24B of the holder 14C is similar to the second tube 24B described with reference to Figs. 7A to 9B. In the holder 14C, the same components as those of the holder 14A or the holder 14B are denoted by the same reference numerals, and detailed description thereof will be omitted.

As shown in Figs. 10A and 10B, the first tube 22C includes a cylindrical large-diameter portion 50C and the proximal wall 51 that closes a proximal end of the large-diameter portion 50C. The large-diameter portion 50C has a cylindrical shape having an outer diameter dimension the same as the head portion 62B of the second tube 24B. The large-diameter portion 50C includes the first stopper 88 and the first adhesion portion 501. The first stopper 88 is a tubular protrusion protruding outward from the large-diameter portion 50C. The first stopper 88 prevents the elastic tube body 500 from moving in the proximal direction.

The first adhesion portion 501 is located on the distal side of the first stopper 88, and is in close contact with the elastic tube body 500.

The proximal wall 51 is configured as a wall surface that closes the proximal end of the large-diameter portion 50C. The proximal wall 51 is jointed to the flange 44A of the needle unit 18A by welding or bonding. A tubular first guide portion 84C extends from the proximal wall 51 toward the distal side in the axial direction. The first guide portion 84C has a cylindrical shape. The first guide portion 84C accommodates a portion of the needle unit 18A on the distal side from the flange 44A therein. In addition, the second guide portion 86 of the second tube 24B is attached to an outer peripheral portion of the first guide portion 84C so as to be slidable in the axial direction. The first guide portion 84C engages with the second guide portion 86 and guides the movement direction of the second tube 24B in the axial direction.

The holder 14C according to the embodiment can reduce the number of components, simplify the structure, and reduce the number of steps required for assembly by directly jointing the first tube 22C and the needle base portion 36A.

### (Fifth Embodiment)

As shown in Fig. 11, a collection kit 10D according to the embodiment is different from the collection kit 10 of Fig. 1 in a liquid accommodation unit 12D. Since the holder 14 of the collection kit 10D is the same as the holder 14 of Fig. 1, the description thereof will be omitted.

As shown in Fig. 11, the liquid accommodation unit 12D includes a collection pipe 122, a connection tube 124, an exhaust port 126, and a discharge port 128. The collection pipe 122 accommodates an amount of the liquid that can be introduced into the two culture bottles 16. The collection pipe 122 is formed of a transparent material such that a liquid surface can be visually recognized. The collection pipe 122 is provided with a scale line (not shown) for checking a collection amount into the culture bottle 16.

The connection tube 124 is attached to an upper portion of the collection pipe 122. The connection tube 124 is connected to a tube for connecting a medical bag by an aseptic jointing device. The liquid in the collection pipe 122 is introduced through the connection tube 124. A clamp 125 may be attached to the connection tube 124.

The exhaust port 126 is attached to the upper portion of the collection pipe 122. The exhaust port 126 discharges internal air when the liquid is introduced into the collection pipe 122.

The discharge port 128 is located at a lower end of the collection pipe 122. The discharge port 128 is a portion that discharges the liquid inside the collection pipe 122. The discharge port 128 is connected to the holder 14.

The collection kit 10D according to the embodiment is configured as described above. Since the collection kit 10D according to the embodiment also includes the holder 14, contamination of the needle tube 34 can be prevented. Therefore, the collection kit 10D can dispense the liquid into the culture bottle 16 without contaminating the liquid.

### (Sixth Embodiment)

As shown in Fig. 12A, a holder 14E according to the embodiment includes a cylindrical first module 30E and a second module 32E. The second module 32E is displaceable in a direction approaching the first module 30E. Note that in the holder 14E, members similar to the members already described with reference to Figs. 1 to 11 are denoted by the same reference numerals, and detailed description thereof will be omitted.

The first module 30E includes the needle unit 18A and a first tube 22E. The needle unit 18A is as described with reference to Fig. 6A. The first tube 22E includes a cylindrical main body 91 and a proximal wall 92 that closes a proximal portion of the main body 91. A distal portion 91a of the main body 91 is an opening portion. The main body 91 accommodates a second tube 24E of the second module 32E through the opening portion. The main body 91 accommodates the second tube 24E slidably in the axial direction along an inner peripheral surface 91b.

As shown in Fig. 12B, the main body 91 has the first chamber 58 inside. The first chamber 58 is surrounded by the inner peripheral surface 91b. The proximal side of the first chamber 58 is closed by the proximal wall 92 and the needle unit 18A, and the distal side of the first chamber 58 is closed by the second tube 24E. The inner peripheral surface 91b comes in contact with the second tube 24E without a gap, and prevents dust and bacteria from entering the first chamber 58.

The main body 91 has a guide groove 91c extending in the axial direction at a predetermined position of the inner peripheral surface 91b in the circumferential direction. The guide groove 91c is engaged with a guide protrusion 96 of the second tube 24E described later, and guides a movement direction of the second tube 24E in the axial direction. The guide groove 91c prevents rotational displacement of the second tube 24E. The main body 91 has a claw portion 91d at a distal end of the guide groove 91c. The claw portion 91d engages with a first concave portion 96a of the guide protrusion 96 to prevent the second tube 24E from being pulled out.

The second module 32E includes the second tube 24E and the lid 28. The second tube 24E includes the head portion 62, the shoulder portion 63, the body portion 64, the partition wall 68, a sliding portion 94, and the guide protrusion 96. Of these, the head portion 62, the shoulder portion 63, the body portion 64, and the partition wall 68 are the same as those in Figs. 2 and 3B. In addition, the partition wall 68 has the through-hole 68a.

As shown in Fig. 12B, the sliding portion 94 has a tubular shape having an outer dimension the same as that of the body portion 64. The sliding portion 94 extends from the body portion 64 toward the proximal side in the axial direction. The sliding portion 94 is formed integrally with the body portion 64. Therefore, an outer peripheral portion of the second tube 24E has a cylindrical shape in which the sliding portion 94 and the body portion 64 are connected without a boundary. The sliding portion 94 is located outside and spaced apart from the head portion 62, and surrounds the head portion 62. The sliding portion 94 is accommodated in the main body 91 of the first tube 22E and slides on the inner peripheral surface 91b.

The second tube 24E includes two guide protrusions 96 at positions separated by 180° in the circumferential direction. The guide protrusion 96 is disposed at a predetermined position of the second tube 24E in the circumferential direction and protrudes outward from the second tube 24E. As shown in Fig. 12A, the guide protrusion 96 is a rib-shaped protrusion that extends linearly in the axial direction. As shown in Fig. 12B, the guide protrusion 96 extends from a proximal end of the sliding portion 94 to a distal end of the body portion 64. The guide protrusion 96 engages with the guide groove 91c of the first tube 22E and slides in the guide groove 91c in the axial direction. The guide protrusion 96 and the guide groove 91c prevent relative rotation between the first tube 22E and the second tube 24E.

The guide protrusion 96 includes the first concave portion 96a and a second concave portion 96b. The first concave portion 96a is located closer to the proximal end, and the second concave portion 96b is located closer to the distal end. The first concave portion 96a engages with the claw portion 91d to prevent the second tube 24E from being pulled out from the first tube 22E. In an initial state of the holder 14E, the first concave portion 96a and the claw portion 91d are engaged with each other. When the second tube 24E is pushed into the first tube 22E, the second concave portion 96b engages with the claw portion 91d and holds the second tube 24E at a position pushed into the first tube 22E.

The holder 14E according to the embodiment is configured as described above. As shown in Fig. 13A, the culture bottle 16 is mounted on the second tube 24E of the holder 14E. Thereafter, when the culture bottle 16 is pushed toward the proximal side, as shown in Fig. 13B, the second tube 24E is displaced toward the proximal end. As a result, the needle tube 34 punctures the rubber stopper 16b of the culture bottle 16, and the culture bottle 16 is connected to the holder 14E.

As described above, a structure of the holder 14E according to the embodiment can be further simplified.

Note that, the invention is not limited to the above-described disclosure, and various configurations can be adopted without departing from the gist of the invention.

## Claims

1. A holder for connecting a culture bottle to a collection kit, the holder comprising:
a needle unit including a needle tube to be punctured into a mouth portion of the culture bottle and a needle base portion that supports the needle tube;
a first tube supported by the needle base portion and including a first chamber that accommodates the needle tube therein; and
a second tube including a second chamber that accommodates the mouth portion of the culture bottle and movable relative to the first tube, wherein
the second tube moves toward the needle unit to cause the needle tube to puncture into the mouth portion of the culture bottle.

2. The holder according to claim 1, further comprising:
a sealing member that seals a gap between the first tube and the second tube and prevents bacteria from entering the first chamber.

3. The holder according to claim 1, further comprising:
a partition wall provided in the second tube to partition the first chamber and the second chamber.

4. The holder according to claim 3, wherein
the partition wall has a through-hole through which the needle tube passes.

5. The holder according to claim 4, wherein
the needle tube is located on a proximal side with respect to the through-hole of the partition wall in an initial state before the culture bottle is attached.

6. The holder according to claim 1, wherein
the second tube includes an engagement protrusion that engages with the mouth portion of the culture bottle accommodated in the second chamber to prevent the culture bottle from being detached from the second tube.

7. The holder according to claim 2, wherein
the second tube includes a sliding portion that slides while overlapping the first tube in a radial direction, and
the sealing member includes a packing that seals the sliding portion.

8. The holder according to claim 2, wherein
the sealing member includes an elastic tube body made of an elastic material that is deflatable in an axial direction, and
the elastic tube body is closely attached to the first tube and the second tube, and the first tube and the second tube are connected via the elastic tube body.

9. The holder according to claim 8, further comprising:
a first guide portion provided in the first tube and protruding toward the second tube; and
a second guide portion provided in the second tube, protruding toward the first tube, and sliding on the first guide portion, wherein
the first guide portion and the second guide portion guide a displacement direction of the second tube in the axial direction.

10. The holder according to claim 1, further comprising:
a lid that covers an opening portion of the second tube, wherein
the lid includes a plate body that covers the opening portion and a fitting wall that is fitted to the second tube to prevent bacteria from entering the first chamber and the second chamber.

11. The holder according to claim 1, further comprising:
a lid that covers an opening portion of the second tube, wherein
the lid includes a sheet body that prevents passage of bacteria, and a joint portion that joints the sheet body to the second tube.

12. The holder according to any one of claims 1 to 11,
wherein
the needle base portion includes
a main body portion extending in the axial direction and jointed to an outer peripheral portion of the needle tube,
a flange protruding outward in a plate shape from the main body portion, and
a first screw formed in the main body portion, and
the first tube is screwed to the first screw and attached to the flange.

13. The holder according to any one of claims 1 to 11, wherein
the needle base portion includes
a main body portion extending in the axial direction and jointed to an outer peripheral portion of the needle tube, and
a flange protruding outward in a plate shape from the main body portion, and
the first tube is jointed to the flange.

14. A collection kit comprising:
a liquid accommodation unit that accommodates a liquid; and
a holder that communicates with the liquid accommodation unit and to which a culture bottle is connected, wherein
the holder includes
a needle unit including a needle tube to be punctured into a mouth portion of the culture bottle and a needle base portion that supports the needle tube,
a first tube supported by the needle base portion and including a first chamber that accommodates the needle tube therein, and
a second tube including a second chamber that accommodates the mouth portion of the culture bottle and movable relative to the first tube, wherein
the second tube moves toward the needle unit to cause the needle tube to puncture into the mouth portion of the culture bottle.
